(19) Europäisches Patentamt
European Patent Office
Office européen des brevets

(11) Veröffentlichungsnummer : **0 146 016**
**B1**

(12) # EUROPÄISCHE PATENTSCHRIFT

(45) Veröffentlichungstag der Patentschrift : **10.09.86**

(51) Int. Cl.[4] : **C 01 B 21/14**

(21) Anmeldenummer : **84114257.3**

(22) Anmeldetag : **26.11.84**

(54) **Stabilisierte Lösungen von Hydroxylamin oder dessen Salzen in Wasser oder Alkoholen sowie deren Herstellung.**

(30) Priorität : **02.12.83 DE 3343599**

(43) Veröffentlichungstag der Anmeldung : **26.06.85 Patentblatt 85/26**

(45) Bekanntmachung des Hinweises auf die Patenterteilung : **10.09.86 Patentblatt 86/37**

(84) Benannte Vertragsstaaten : **BE CH DE FR GB IT LI NL**

(56) Entgegenhaltungen :
**CHEMICAL ABSTRACTS, Band 80, 1974, Seite 111 Nr. 72560g, Columbus, Ohio, US**

(73) Patentinhaber : **BASF Aktiengesellschaft**
**Carl-Bosch-Strasse 38**
**D-6700 Ludwigshafen (DE)**

(72) Erfinder : **Grosskinsky, Otto-Alfred, Dr. Chem.**
**Semmelweisstrasse 8**
**D-6700 Ludwigshafen (DE)**
Erfinder : **Frommer, Elmar, Dr. Chem.**
**Lisztstrasse 117**
**D-6700 Ludwigshafen (DE)**
Erfinder : **Ritz, Josef, Dr. Chem.**
**Osloer Weg 8**
**D-6700 Ludwigshafen (DE)**
Erfinder : **Thomas, Erwin**
**Borngasse 12**
**D-6713 Freinsheim (DE)**
Erfinder : **Weiss, Franz-Josef, Dr. Chem.**
**Schilfweg 1**
**D-6708 Neuhofen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 146 016 B1

Jouve, 18, rue St-Denis, 75001 Paris, France

**Beschreibung**

Lösungen von Hydroxylammoniumsalzen zersetzen sich langsam bei Raumtemperatur und schneller bei erhöhter Temperatur. Dies gilt in vermehrtem Maße für Lösungen von freiem Hydroxylamin. Es hat nicht an Versuchen gefehlt, Lösungen von Hydroxylamin und dessen Salzen zu stabilisieren, um eine verbesserte Lagerfähigkeit zu erzielen. So verwendet man als Stabilisierungsmittel Harnstoffderivate entsprechend der US-PS 3 544 270. Aus der US-PS 3 480 391 ist bekannt, daß sich Amidoxime als Stabilisatoren eignen. Entsprechend der US-PS 3 480 392 werden hierfür Hydroxamsäuren empfohlen. Aus der US-PS 3 145 082 ist auch schon bekannt, chelatbildende Mittel wie das Natriumsalz der Ethylendiamintetraessigsäure als Stabilisierungsmittel zu verwenden. Die bislang verwendeten Stabilisierungsmittel sind noch verbesserungsbedürftig.

Es war die technische Aufgabe gestellt, stabilisierte Lösungen von Hydroxylamin oder dessen Salzen zur Verfügung zu stellen, die über einen längeren Zeitraum stabil sind und insbesondere die Zersetzung von freiem Hydroxylamin minimiert wird.

Diese Aufgabe wird gelöst durch stabilisierte Lösungen von Hydroxylamin oder dessen Salzen in Wasser oder Alkoholen, gekennzeichnet durch einen Gehalt an Flavanen der Formel

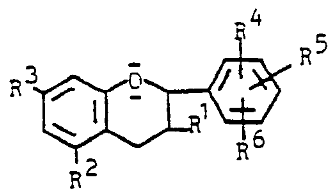

(I)

in der $R^1$ bis $R^6$ jeweils ein Wasserstoffatom, eine Hydroxylgruppe, eine $C_1$- bis $C_4$-Alkoxygruppe oder eine $C_1$- bis $C_4$-Acyloxygruppe bezeichnen, mit der Maßgabe, daß mindestens ein Substituent eine Hydroxylgruppe ist.

Ferner ist ein Gegenstand der Erfindung ein Verfahren zur Herstellung von stabilisierten Lösungen von Hydroxylamin oder dessen Salzen durch Zugabe von Stabilisatoren, dadurch gekennzeichnet, daß man aus der zu stabilisierenden Lösung den darin gelöst enthaltenen molekularen Sauerstoff durch Behandeln mit Stickstoff, der frei von molekularem Sauerstoff ist, entfernt und dann Flavane, die mindestens eine Hydroxylgruppe enthalten, zusetzt.

Die gemäß der Erfindung stabilisierten Lösungen von Hydroxylamin oder dessen Salzen haben den Vorteil, daß sie über längere Zeit als bisher stabil sind und insbesondere die Zersetzung von freiem Hydroxylamin auf ein Minimum reduziert wird.

Erfindungsgemäß geht man von Lösungen von Hydroxylamin oder dessen Salzen in Wasser oder Alkoholen, z. B. $C_1$- bis $C_4$-Alkanolen, aus. Geeignete Salze des Hydroxylamins sind beispielsweise solche mit starken Mineralsäuren, wie Schwefelsäure, Salpetersäure, Chlorwasserstoffsäure oder solche mit Fettsäuren, z. B. Essigsäure oder Propionsäure. Aufgrund der verschiedenen Löslichkeiten liegt Hydroxylamin vorzugsweise gelöst in Wasser oder Alkoholen vor, während dessen Salze vorzugsweise als wäßrige Lösungen vorliegen. Der Gehalt an Hydroxylamin oder dessen Salzen beträgt in der Regel von 10 bis 70 Gew.-%. Die Ausgangslösungen haben in der Regel einen pH-Wert von 8 bis 11. Besonders bevorzugt geht man von Lösungen von Hydroxylamin in Wasser aus.

Als Stabilisatoren verwendet man Flavane, die mindestens eine Hydroxylgruppe, insbesondere mindestens drei Hydroxylgruppen im Molekül enthalten. Bevorzugte Flavane sind solche der Formel

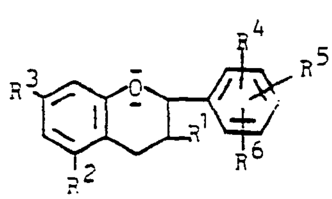

(I)

in der $R^1$ bis $R^6$ jeweils ein Wasserstoffatom, eine Hydroxylgruppe, eine $C_1$- bis $C_4$-Alkoxygruppe oder eine $C_1$- bis $C_4$-Acyloxygruppe bezeichnet, mit der Maßgabe, daß mindestens ein Substituent eine Hydroxylgruppe ist. In besonders bevorzugten Flavanen der Formel (I) bezeichnen $R^1$ bis $R^6$ jeweils ein Wasserstoffatom, eine Hydroxylgruppe oder eine Methoxygruppe, mit der Maßgabe, daß mindestens drei Substituenten eine Hydroxylgruppe bedeuten. Die Hydroxylgruppen enthaltenden Flavane können auch als Glykoside vorliegen. Geeignete Flavane sind aus Angewandte Chemie *68* (1956), Seiten 110 und 111 bekannt.

Vorteilhaft wendet man die Hydroxylgruppen enthaltenden Flavane in Mengen von 0,005 bis 1 Gew.%, insbesondere 0,01 bis 0,1 Gew.-%, bezogen auf die zu stabilisierende Lösung an. Es hat sich ferner

bewährt, wenn man zusätzlich Polyhydroxybenzole, insbesondere Pyrogallol mitverwendet. Polyhydroxybenzole werden vorteilhaft in Mengen von 0,005 bis 0,1 Gew.%, bezogen auf die zu stabilisierende Lösung zugesetzt. Es ist bemerkenswert, daß durch die kombinierte Anwendung von Hydroxylgruppen enthaltenden Flavanen und Polyhydroxybenzolen eine synergistische Wirkung erzielt wird.

Stabilisierte Lösungen von Hydroxylamin oder dessen Salzen in Wasser oder Alkoholen werden erfindungsgemäß hergestellt, indem man aus den zu stabilisierenden Lösungen zunächst den darin gelöst enthaltenen molekularen Sauerstoff durch Behandeln mit Stickstoff, der frei von molekularem Sauerstoff ist, verdrängt. Dies erzielt man beispielsweise dadurch, daß man durch die zu stabilisierende Lösung sauerstoffreien Stickstoff leitet, z. B. für 5 bis 10 Minuten. Der angewandte Stickstoff enthält vorteilhaft weniger als 2 ppm molekularen Sauerstoff. Dann gibt man Hydroxylgruppen enthaltende Flavane und gegebenenfalls Polyhydroxybenzole zu und löst diese in der zu stabilisierenden Lösung auf. Hierbei hält man vorteilhaft eine Temperatur von 5 bis 40 °C ein. Es ist auch möglich, die Stabilisatoren bereits in gelöstem Zustand, z. B. gelöst in $C_1$- bis $C_4$-Alkanolen den zu stabilisierenden Lösungen zuzugeben.

Es versteht sich, daß es von Vorteil ist, eine Kontamination von zu stabilisierenden Lösungen mit Schwermetallen, insbesondere Kupfer oder Edelmetallen zu vermeiden, da diese die Zersetzung von Hydroxylamin katalysieren. Ferner ist es von Vorteil, energiereiche Strahlen durch geeignet eingefärbte Glasbehälter auszuschalten. Schließlich ist es von Vorteil, die stabilisierten Lösungen bei Temperaturen von $< 40$ °C, z. B. bei Temperaturen von 5 bis 20 °C aufzubewahren.

Stabilisierte Lösungen von Hydroxylamin oder dessen Salze eignen sich zur Herstellung von Oximen. Der erfindungsgemäße Gegenstand sei an folgendem Beispiel veranschaulicht.

## Beispiel

Eine wäßrige Lösung von Hydroxylamin wird bei 20 °C 10 Minuten mit sauerstoffreiem Stickstoff begast und dann der Stabilisator zugefügt. Die Konzentration an Hydroxylamin, die zugegebene Menge und Art des Stabilisators sowie die in Abhängigkeit von Zeit und Temperatur erzielten Ergebnisse sind aus folgender Tabelle ersichtlich.

Tabelle

| Stabilisator | °C | | | | |
|---|---|---|---|---|---|
| 50 ppm d-Catechin | 5 | 0 | 743 | 1198 | Stunden |
| | | 110,88 | 110,72 | 110,55 | g/l $NH_2OH$ |
| 50 ppm d-Catechin | 20 | 0 | 404 | 1195 | Stunden |
| | | 110,25 | 109,63 | 109,49 | g/l $NH_2OH$ |
| 50 ppm d-Catechin | 40 | 0 | 359 | 1194 | Stunden |
| | | 110,25 | 109,79 | 108,57 | g/l $NH_2OH$ |

## Patentansprüche

1. Stabilisierte Lösungen von Hydroxylamin oder dessen Salzen in Wasser oder Alkoholen, gekennzeichnet durch einen Gehalt an Flavanen der Formel

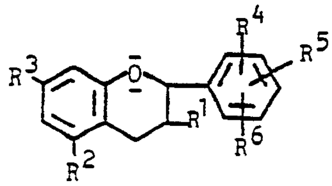

(I)

in der $R^1$ bis $R^6$ jeweils ein Wasserstoffatom, eine Hydroxylgruppe, eine $C_1$- bis $C_4$-Alkoxygruppe oder eine $C_1$- bis $C_4$-Acyloxygruppe bezeichnen, mit der Maßgabe, daß mindestens ein Substituent eine Hydroxylgruppe ist.

2. Stabilisierte Lösungen nach Anspruch 1, dadurch gekennzeichnet, daß in den Flavanen der Formel (I) $R^1$ bis $R^6$ jeweils ein Wasserstoffatom, eine Hydroxylgruppe oder eine Methoxygruppe bezeichnen, mit der Maßgabe, daß mindestens drei Substituenten eine Hydroxylgruppe bedeuten.

3. Stabilisierte Lösungen nach den Ansprüchen 1 und 2, gekennzeichnet durch eine Gehalt von 0,005 bis 1 Gew.-% Flavanen der Formel (I), bezogen auf die zu stabilisierende Lösung.

4. Stabilisierte Lösungen nach den Ansprüchen 1 bis 3, gekennzeichnet durch durch einen zusätzlichen Gehalt an Pyrogallol.

5. Verfahren zur Herstellung von stabilisierten Lösungen von Hydroxylamin oder dessen Salze in Wasser oder Alkoholen durch Zusatz von Stabilisatoren, dadurch gekennzeichnet, daß man aus den zu stabilisierenden Lösungen den darin gelöst enthaltenen molekularen Sauerstoff durch Behandeln mit Stickstoff, der frei von molekularem Sauerstoff ist, entfernt.und dann Hydroxylgruppen enthaltende Flavane zusetzt.

## Claims

1. A stabilized solution of hydroxylamine or its salts in water or an alcohol, which contains a flavan of the formula

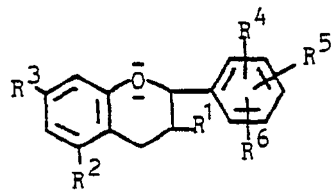

(I)

where $R^1$, $R^2$, $R^3$, $R^4$, $R^5$ and $R^6$ are each hydrogen, hydroxyl, $C_1$-$C_4$-alkoxy or $C_1$-$C_4$-acyloxy, with the proviso that at least one of the substituents is hydroxyl.

2. A stabilized solution as claimed in claim 1, wherein, in the flavan of the formula (I), $R^1$, $R^2$, $R^3$, $R^4$, $R^5$ and $R^6$ are each hydrogen, hydroxyl or methoxy, with the proviso that at least three of the substituents are hydroxyl.

3. A stabilized solution as claimed in claims 1 and 2, which contains from 0.005 to 1 % by weight, based on the solution to be stabilized, of a flavan of the formula (I).

4. A stabilized solution as claimed in claims 1 to 3, which additionally contains pyrogallol.

5. A process for the preparation of a stabilized solution of hydroxylamine or its salts in water or an alcohol, wherein the molecular oxygen dissolved in the solution to be stabilized is removed from this solution by treatment with nitrogen which is free of molecular oxygen, and a hydroxyl-containing flavan is then added.

## Revendications

1. Solutions stabilisées d'hydroxylamine, ou de ses sels, dans de l'eau ou des alcools, caractérisées par une teneur en flavanes de formule

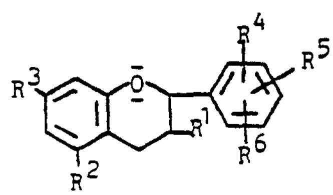

(I)

dans laquelle $R^1$ à $R^6$ représentent respectivement un atome d'hydrogène, un groupe hydroxyle, un groupe alcoxy en $C_1$ à $C_4$ ou un groupe acyloxy en $C_1$ à $C_4$, sous réserve qu'au moins un substituant est un groupe hydroxyle.

2. Solutions stabilisées selon la revendication 1, caractérisées par le fait que, dans les flavanes de formule I, $R^1$ à $R^6$ représentent respectivement un atome d'hydrogène, un groupe hydroxyle ou un groupe méthoxy, sous réserve qu'au moins un substituant est un groupe hydroxyle.

3. Solutions stabilisées selon les revendications 1 et 2, caractérisées par une teneur de 0,005 à 1 % en poids de flavanes de formule I, rapportée à la solution stabilisée.

4. Solutions stabilisées selon les revendications 1 à 3, caractérisées par une teneur additionnelle en pyrogallol.

5. Procédé de préparation de solutions stabilisées d'hydroxylamine, ou de ses sels, dans de l'eau ou des alcools, par addition de stabilisants, caractérisé par le fait que l'on élimine des solutions à stabiliser l'oxygène moléculaire qui y est dissous, par traitement avec de l'azote, qui est exempt d'oxygène moléculaire, puis on ajoute des flavanes contenant des groupes hydroxyle.